Europäisches Patentamt

European Patent Office

Office européen des brevets

(11) Veröffentlichungsnummer : **0 041 591**
**B1**

(12)

# EUROPÄISCHE PATENTSCHRIFT

(45) Veröffentlichungstag der Patentschrift :
07.12.83

(51) Int. Cl.³ : **A 61 F   1/03**

(21) Anmeldenummer : **81100771.5**

(22) Anmeldetag : **04.02.81**

(54) Gelenkendoprothese und Scheibenkörper als Drehsicherung.

(30) Priorität : **09.05.80 CH 3647/80**

(43) Veröffentlichungstag der Anmeldung :
**16.12.81 Patentblatt 81/50**

(45) Bekanntmachung des Hinweises auf die Patenterteilung : **07.12.83 Patentblatt 83/49**

(84) Benannte Vertragsstaaten :
**AT DE FR GB IT NL**

(56) Entgegenhaltungen :
DE-A- 2 305 442
FR-A- 1 590 649
FR-A- 2 398 490
FR-A- 2 412 304
US-A- 3 228 393
US-A- 3 670 724
US-A- 3 765 034

(73) Patentinhaber : **Protek AG**
**Stadtbachstrasse 64**
**CH-3000 Bern (CH)**

(72) Erfinder : **Niederer, Peter Gino, Dipl.-Ing. ETH**
**Reichenbachstrasse 6**
**CH-3052 Zollikofen (CH)**   ·

(74) Vertreter : **Dipl.-Ing. H. Marsch Dipl.-Ing. K. Sparing**
**Dipl.-Phys. Dr. W.H. Röhl Patentanwälte**
**Rethelstrasse 123**
**D-4000 Düsseldorf (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Gelenkendoprothese und Scheibenkörper als Drehsicherung

Die Erfindung betrifft eine Gelenkendoprothese mit Drehsicherung, wobei im proximalen Bereich des Prothesenschaftes mindestens ein Durchbruch vorhanden ist. Ausserdem betrifft sie einen Scheibenkörper als Drehsicherung. Abgesehen von den unbeabsichtigten Drehungen, die rotations-symmetrische Schäfte, beispielsweise mit kreisförmigem Querschnitt, im Knochen nach dem Einsetzen ausführen, treten solche Drehungen vor allem auch bei blattartigen Schäften auf, die beispielsweise in der DE-A1-27 46 664 gezeigt und beschrieben sind. Aus der US-A-3, 228,393 ist ein blattartiger Schaft bekannt, der mit Durchbrüchen versehen ist; in diese werden Knochentransplantate eingeschoben und mit Hilfe von Keilnuten gegen Verschiebungen und Verdrehungen gesichert.

Bekanntlich ist jedoch das proximale Ende des Femurknochens, das beispielsweise einen blattartigen Schaft einer Hüftgelenkprothese aufnimmt, von der Seite gesehen S-förmig ausgebildet und weist zwei verschiedene Krümmungen auf, nämlich die Ante-Torsion des Schenkelhalses (Drehung nach vorn) und die Ante-Kurvation der Femur-«Röhre» (Biegung mit der Konvexität nach vorn). Dadurch wird ein gerader oder auch leicht gebogener Gegenstand, wie ein Prothesenschaft, beim Einsetzen von der Wand des Oberschenkelknochens leicht abgelenkt; der mit fest verankerten Knochen-Querrippen versehene Schaft erfährt dabei unerwünschte Verdrehungen, weil sich die Knochentransplantate nicht an die Form der Operationsöffnung anpassen können.

Zwar ist der Querschnitt der kortikalen Begrenzung im Innenraum eines Femurknochens, an der sich der blattartige Prothesenschaft durch Verklemmen abstützen sollte, näherungsweise ein Dreieck; er besitzt jedoch unregelmässige Durchmesser, wie Fig. 5 und 6 der Zeichnungen verdeutlichen. Die Forderung, dass die Blattseite bei einem blattartigen Schaft parallel zur Querachse des Kniegelenks verlaufen sollte, um einerseits eine genormte Implantation zu ermöglichen und andererseits eine ideale Stellung des Gelenkkopfes der Prothese zur ebenfalls ersetzten Gelenkpfanne zu gewährleisten, führt daher sehr häufig dazu, dass die blattartigen Schäfte — wie ebenfalls Fig. 6 erkennen lässt — in dem Holraum innerhalb der kortikalen Schale des Knochens nicht in Richtung des grössten Durchmessers verlaufen; sie sind daher ganz allgemein, d. h. auch ohne darin fest verankerte Querrippen, gegen eine unbeabsichtigte Rotation sehr anfällig, da sie sich dieser gegenüber gewissermassen in einem «labilen» Gleichgewichtszustand befinden.

Aufgabe der Erfindung ist es, eine Gelenkendoprothese mit einer Drehsicherung zu schaffen, die während und nach dem Einschlagen eine unbeabsichtigte Drehung des Prothesenschaftes, insbesondere bei rotationssymmetrischen und bei blattartigen Verankerungsschäften, verhindert, während des Einschlagens der Prothese in den Knochen jedoch relativ zu deren Schaft verschiebbar ist. Gemäss der vorliegenden Erfindung wird diese Aufgabe dadurch gelöst, dass die Drehsicherung ein trapezförmiger Scheibenkörper ist, der in dem Durchbruch verschiebbar gelagert ist, und dessen aufeinander zulaufenden Trapezseiten durch lappenartige Vorsprünge und dazwischen liegende Einschnitte verformbar ausgebildet sind. Der Einsatz der erfindungsgemässen Drehsicherung ist dabei sowohl bei zementfreier oder zementarmer — d. h. nur teilweiser Ummantelung des Schaftes mit Knochenzement — als auch bei einer Verankerung mit einem vollständigen Zementbett möglich.

Der in den Durchbrüchen gelagerte Scheibenkörper kann sich beim Einschlagen der Prothese einerseits in seiner Durchsteckrichtung verschieben, so dass, wenn in einem Schaft mehrere Scheibenkörper vorgesehen sind, diese unter Umständen in der einen oder anderen Richtung verschieden weit aus dem Schaft herausragen und sich so der relativen Lage des Schaftes in der Operationsöffnung anpassen können. Andererseits erfahren die Scheibenkörper an ihren «ausgefransten» Seiten eine Verformung beim Eintreiben des Schaftes in den Hohlraum der kortikalen Knochenschale; die dabei auftretenden Verformungen der lappenartigen Vorsprünge rufen einen Widerstand gegen weitere Verschiebungen hervor. Die Sicherung gegen Verdrehungen wird vor allem durch Anliegen der Kanten des Scheibenkörpers an der Begrenzung der unregelmässig geformten Operationsöffnung bewirkt.

Obwohl die Scheibenkörper aus allen in der Implantattechnik verwendeten und bekannten Werkstoffen gefertigt sein können, werden sie bevorzugt aus Kunststoff, insbesondere aus Polyäthylen, hergestellt. Für solche Kunststoff-Scheibenkörper hat sich als zweckmässig erwiesen, wenn die Länge der Vorsprünge bzw. die Tiefe der Einschnitte zur minimalen Dicke der Vorsprünge in einem Verhältnis von 4 : 1 bis 5 : 1 stehen. Mit diesen Bemessungen werden sowohl eine ausreichende Verformung der aus den Schäften herausragenden Enden der Scheibenkörper bei Einsetzen des Schaftes, als auch genügend grosse Widerstandskräfte gegen unbeabsichtigte Verschiebungen und Verdrehungen erreicht.

Im folgenden wird die Erfindung anhand eines Ausführungsbeispiels näher erläutert.

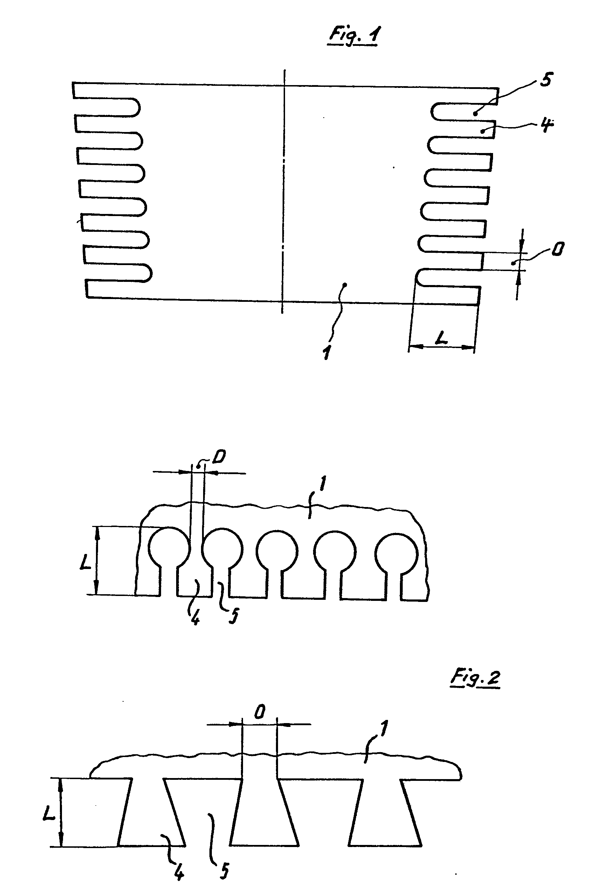

Figur 1 stellt eine Seitenansicht der erfindungsgemässen Drehsicherung dar;

Figur 2 gibt von Fig. 1 abweichende Ausbildung der lappenartigen Vorsprünge an den Trapezseiten des Scheibenkörpers wieder;

Figuren 3 und 4 zeigen eine Ansicht auf eine und eine Ansicht senkrecht zu einer Blattseite eines blattartigen Schaftes mit zwei in seine Durchbrüche eingesteckten Scheibenkörper, während

Figuren 5 und 6 schliesslich Querschnitte durch Femurknochen sind, in die ein blattartiger bzw. ein rotationssymmetrischer Schaft eingesetzt und durch die neuen Scheibenkörper gegen Verdrehen gesichert sind.

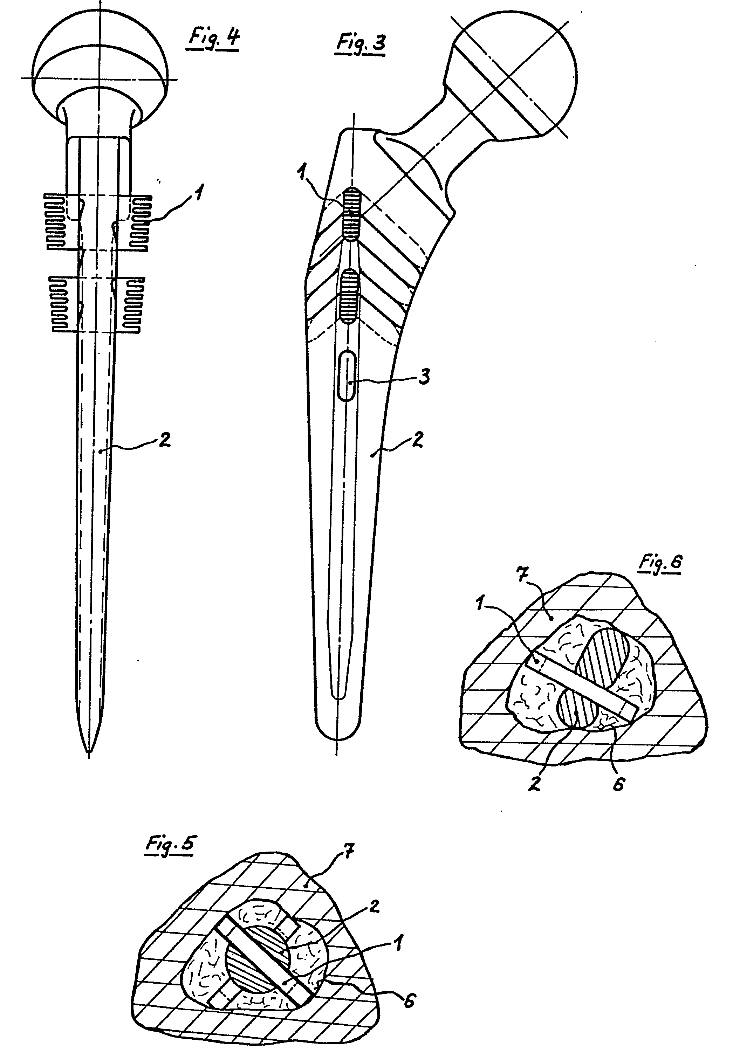

Der Scheibenkörper 1, der vorzugsweise aus Kunststoff, in erster Linie aus Polyäthylen, gefertigt wird, hat die Form eines gleichseitigen Trapezes. Sein Querschnitt, der beispielsweise aus Fig. 3 erkennbar ist, ist an die Durchbrüche 3 in den Schäften 2 (Fig. 3) derart angepasst, dass er, wie bereits erwähnt, beim Einschlagen der Prothese in diesen Durchbrüchen 3 verschiebbar ist.

Erfindungsgemäss sind die aufeinander zulaufenden Trapezseiten mit lappenartigen Vorsprüngen 4 und dazwischen liegenden Einschnitten 5 versehen, so dass sich die lappenartigen Vorsprünge 4 beim Einschlagen des Schaftes 2, wie geschildert, verformen und an den Wänden 6 (Fig. 5 und 6) der kortikalen Schale 7 des Knochens verklemmen.

Wie experimentell ermittelt worden ist, ergeben sich für aus Kunststoff gefertigte Scheibenkörper 1 besonders günstige Verhältnisse zwischen Verformbarkeit und Widerstand gegen unbeabsichtigte Drehungen oder Verschiebungen, wenn die Länge L der Vorsprünge 4 etwa 4 bis 6 mal grösser ist als ihre Dicke D an ihrer engsten Stelle.

**Ansprüche**

1. Gelenkendoprothese mit Drehsicherung, wobei im proximalen Bereich des Prothesenschaftes (2) mindestens ein Durchbruch (3) vorhanden ist, dadurch gekennzeichnet, dass die Drehsicherung ein trapezförmiger Scheibenkörper (1) ist, der in dem Durchbruch (3) verschiebbar gelagert ist, und dessen aufeinander zulaufenden Trapezseiten durch lappenartige Vorsprünge (4) und dazwischen liegende Einschnitte (5) verformbar ausgebildet sind.

2. Scheibenkörper nach Anspruch 1, der aus Kunststoff, insbesondere aus Polyäthylen besteht, dadurch gekennzeichnet, dass die Länge (L) der Vorsprünge (4) bzw. die Tiefe der Einschnitte (5) zur minimalen Dicke (D) der Vorsprünge (4) in einem Verhältnis von 4 : 1 bis 5 : 1 stehen.

**Claims**

1. A joint endoprothesis comprising a rotation-preventing means, the proximal region of the prothesis stem (2) being formed with at least one aperture (3), characterised in that the rotation-preventing means is a trapezoidal plate member (1) movably mounted in the aperture (3) and the converging sides of the trapezoid are made deformable by means of lug-like projections (4) and recesses (5) lying therebetween.

2. A plate member according to claim 1 made of plastics, more particularly polythene, characterised in that the length (L) of the projections (4) and the depth of the recesses (5) is in a proportion of 4 : 1 to 5 : 1 to the minimum thickness (D) of the projections (4).

**Revendications**

1. Endoprothèse d'articulation avec blocage de rotation, au moins un percement (3) étant présent dans la zone proche de la tige de prothèse (2), caractérisée en ce que le blocage en rotation est un corps formant plaque (1), trapézoïdal, qui est monté dans le percement (3) de manière à pouvoir se déplacer et dont les côtés de trapèze convergeant l'un vers l'autre sont réalisés avec possibilité de déformation par des protubérances (4) du type languette et par des encoches intermédiaires (5).

2. Corsp formant plaque selon la revendication 1, qui est constitué par de la matière plastique, en particulier par du polyéthylène, caractérisé en ce que le longueur (L) des protubérances (4) ou la profondeur des encoches (5) par rapport à l'épaisseur minimale (D) des protubérances (4) se trouvent dans un rapport de 4 : 1 à 5 : 1.

3

Fig. 1

Fig. 2

Fig. 4

Fig. 3

Fig. 6

Fig. 5